(19) Europäisches Patentamt
European Patent Office
Office européen des brevets

(11) EP 0 895 471 B1

(12) EUROPÄISCHE PATENTSCHRIFT

(45) Veröffentlichungstag und Bekanntmachung des Hinweises auf die Patenterteilung:
**02.04.2003 Patentblatt 2003/14**

(21) Anmeldenummer: **97909330.9**

(22) Anmeldetag: **29.09.1997**

(51) Int Cl.[7]: **A61K 7/13**

(86) Internationale Anmeldenummer:
**PCT/EP97/05336**

(87) Internationale Veröffentlichungsnummer:
**WO 98/019659 (14.05.1998 Gazette 1998/19)**

(54) **FÄRBEMITTEL ZUR FÄRBUNG VON KERATINISCHEN FASERN**

DYEING AGENT FOR DYEING KERATIN FIBRES

AGENT ET PROCEDE POUR LA COLORATION DE FIBRES KERATINIQUES

(84) Benannte Vertragsstaaten:
**DE ES FR GB IT**

(30) Priorität: **05.11.1996 DE 19645540**

(43) Veröffentlichungstag der Anmeldung:
**10.02.1999 Patentblatt 1999/06**

(73) Patentinhaber: **Wella Aktiengesellschaft**
**64274 Darmstadt (DE)**

(72) Erfinder: **BRAUN, Hans-Jürgen, Dr.**
**3182 Überstorf (CH)**

(56) Entgegenhaltungen:
**DE-A- 3 834 142** **DE-A- 3 942 294**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 895 471 B1

Printed by Jouve, 75001 PARIS (FR)

## Beschreibung

Die vorliegende Erfindung beschreibt eine Zusammensetzung zur Färbung von keratinischen Fasern, insbesonders von menschlichen Haaren, mit einem Gehalt an einem am Benzolring monosubstituierten p-Phenylendiamin, einem substituierten m-Phenylendiamin und einem 2-Amino-6-chlor-4-nitrophenol-Derivat.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines Oxidationsmittels.

An Oxidationsfarbstoffe, die zur Färbung menschlicher Haare verwendet werden sollen, sind zahlreiche besondere Anforderungen gestellt. Die Farbstoffe müssen in toxikologischer und dermatologischer Hinsicht unbedenklich sowie nicht sensibilisierend sein und Färbungen in der gewünschten Intensität ermöglichen.

Ferner wird für die erzielten Haarfärbungen eine gute Licht-, Dauerwell-, Säure- und Reibeechtheit gefordert. Die Haarfärbungen sollen auch ohne Einwirkung von Licht, Reibung und chemischen Mitteln über einen Zeitraum von mindestens vier bis sechs Wochen stabil bleiben.

Außerdem ist es erforderlich, daß durch Kombination geeigneter Entwickler- und Kupplerkomponenten eine breite Palette verschiedener Farbnuancen erzeugt werden kann.

Ein weiteres Problem besteht in der Praxis bezüglich des unterschiedlichen Aufziehverhaltens der Farbstoffe in Abhängigkeit von der Haarbeschaffenheit, wodurch eine ungleichmäßige Anfärbung der Haare erfolgt. Normalerweise neigen die verwendeten Farbstoffe dazu, auf geschädigte Haarpartien stärker aufzuziehen als auf ungeschädigte Haarpartien. Aus diesem Grunde werden in der Regel die durch übliche Alterungsprozesse und Umwelteinflüsse (zum Beispiel Sonnenlicht, Haarwäsche, Färbe- und Dauerwellbehandlungen) stärker geschädigten Haarspitzen intensiver gefärbt als die weniger geschädigten Haarlängen und der Haaransatz, wodurch ein unnatürliches, ungleichmäßiges und völlig unbefriedigendes Färbeergebnis erhalten wird. Dieses ungleichmäßige Färbeergebnis macht sich insbesondere bei hellen Nuancen sehr störend bemerkbar.

Es bestand daher die Aufgabe, ein Oxidationshaarfärbemittel zur Verfügung zu stellen, das eine gleichmäßige Färbung der Haare von den Haarspitzen bis zum Haaransatz im Naturtonbereich ermöglicht, und gleichzeitig wenig oder gar nicht mutagen und sensibilisierend sowie physiologisch gut verträglich ist.

In der EP-B 0 665 005 wird die Kombination von einem am Benzolring monosubstituierten p-Phenylendiamin und einem substituierten m-Phenylendiamin zur Färbung von Haaren empfohlen. Diese Kombination ergibt jedoch auf unterschiedlich geschädigten Haaren ein unbefriedigendes Farbresultat. Außerdem sind helle Nuancen damit sehr schwierig nuancierbar.

Es wurde nun überraschenderweise gefunden, daß die vorgenannten Nachteile vermieden werdenkönnen, wenn die oxidative Haarfärbung mit einer Kombination von

- mindestens einem p-Phenylendiaminderivat, das ausgewählt ist aus
  2-Hydroxymethyl-p-phenylendiamin
  2-(2',5'-Diaminophenyl)ethanol und
  2-(2'-Hydroxyethoxy)-p-phenylendiamin,
- mindestens einem m-Phenylendiaminderivat, das ausgewählt ist aus
  2-(2',4'-Diaminophenoxy)ethanol,
  1,3-Bis-(2',4'-diaminophenoxy)-propan,
  Bis-(2',4'-diaminophenoxy)methan,
  2-Amino-4-((2'-hydroxyethyl)-amino)-anisol,
  1-(2'-Aminoethoxy)-2,4-diaminobenzol,
  2-(2'-Hydroxyethoxy)-5-(methylamino)anilin,
  2,4-Diamino-1,5-bis-(2'-hydroxyethoxy)benzol und
  1-(2',3'-Dihydroxypropoxy)-2,4-diaminobenzol,
  und
- mindestens einem 2-Amino-6-Chlor-4-nitrophenol der allgemeinen Formel (I),

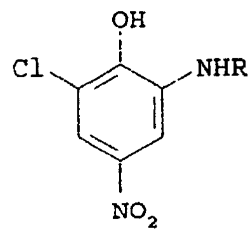

I

,

worin R Wasserstoff, eine geradkettigen oder verzweigte Alkylgruppe mit 1 bis 6 Kohlenstoffatomen, eine Hydroxyalkylgruppe mit 2 bis 4 Kohlenstoffatomen oder eine geradkettige oder verzweigte Polyhydroxyalkylgruppe mit 3 bis 4 Kohlenstoffatomen bedeutet,
durchgeführt wird.

Bevorzugte Derivate gemäß allgemeiner Formel (I) sind
2-Amino-6-chlor-4-nitrophenol, 6-Chlor-2-ethylamino-4-nitrophenol und
6-Chlor-2-((2'-hydroxyethyl)amino)-4-nitrophenol.

Die beschriebenen Farbstoffkombinationen ermöglichen eine vom Haaransatz bis zur Haarspitze gleichmäßige Färbung, ganz besonders auch bei helleren Nuancen. Die vorzüglichen Eigenschaften der neuen Färbstoffkombination zeigen sich insbesondere auf durch Licht und Wetter geschädigtem oder auf dauergewellten Haaren.

Zur Abrundung des Farbergebnisses sowie zur Erzeugung von speziellen Farbeffekten können dem Haarfärbemittel weitere Oxidationsfarbvorstufen wie Resorcin, 4-Chlorresorcin und Derivate des m-Aminophenols sowie direktziehende Farbstoffe, wie zum Beispiel Azofarbstoffe, Anthrachinone oder Nitrobenzolderivate zugesetzt werden.

Die vorstehend beschriebenen erfindungsgemäßen Kombinationen von oxidativen Haarfarbvorstufen und gegebenenfalls direktziehenden Farbstoffen werden zur Färbung in einer geeigneten Farbträgermasse appliziert.

Der Gegenstand der vorliegenden Anmeldung betrifft daher auch ein Mittel zur oxidativen Färbung von Haaren, welches durch Vermischen einer Farbträgermasse mit einem Oxidationsmittel unmittelbar vor der Anwendung hergestellt wird.

Die erfindungsgemäße Farbträgermasse enthält die vorstehend beschriebenen erfindungsgemäßen Kombinationen als solche oder in Form der physiologisch verträglichen Salze, beispielsweise der Hydrochloride, Sulfate oder Tartrate oder im Fall von Phenolen der Alkaliphenolate.

Die Gesamtkonzentration an Farbvorstufen in der Farbträgermasse beträgt 0,1 bis 10 Gewichtsprozent, vorzugsweise 0,2 bis 6 Gewichtsprozent.

Darüberhinaus können in der Farbträgermasse übliche kosmetische Zusätze, beispielsweise Antioxidantien wie Ascorbinsäure, Thioglykolsäure oder Natriumsulfit; Parfümöle; Komplexbildner; Netzmittel und Emulgatoren; Verdicker; Pflegestoffe und andere vorhanden sein.

Die Zubereitungsform für die Farbträgermasse sowie für das gebrauchsfertige Oxidationshaarfärbemittel kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, n-Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 25 Gewichtsprozent (bezogen auf die Farbträgermasse) und die Pflegestoffe in einer Konzentration von etwa 0,1 bis 5,0 Gewichtsprozent (bezogen auf die Farbträgermasse).

Das gebrauchsfertige erfindungsgemäße Haarfärbemittel wird durch Mischen der Farbträgermasse mit einem flüssigen Oxidationsmittel unmittelbar vor der Anwendung hergestellt.

Die Farbträgermasse und das Oxidationsmittel werden hierbei in einem Gewichtsverhältnis von 5:1 bis 1:3

miteinander vermischt, wobei ein Gewichtsverhältnis von 1:1 bis 1:2 besonders bevorzugt ist.

Der pH-Wert des gebrauchsfertigen erfindungsgemäßen Haarfärbemittels stellt sich bei der Mischung der vorzugsweise alkalisch eingestellten Farbträgermasse mit dem in der Regel sauer eingestellten Oxidationsmittel auf einen Wert ein, der durch die Alkalimenge in der Farbträgermasse und die Säuremenge im Oxidationsmittel sowie durch das Mischungsverhältnis bestimmt wird. Der pH-Wert des gebrauchsfertigen Haarfärbemittels beträgt in der Regel 3 bis 11, vorzugsweise 5 bis 9.

Für die Einstellung des jeweiligen pH-Wertes der Farbträgermasse und des Oxidationsmittels können je nach dem gewünschten pH-Wert organische und anorganische Säuren, wie zum Beispiel Phosphorsäure, Ascorbinsäure und Milchsäure, oder Alkalien, wie zum Beispiel Monoethanolamin, Triethanolamin, 2-Amino-2-methyl-1-propanol, Ammoniak, Natronlauge, Kalilauge oder Tris(hydroxymethyl)-amino-methan, verwendet werden.

Für die Anwendung zur oxidativen Färbung von Haaren vermischt man die vorstehend beschriebene Farbträgermasse unmittelbar vor dem Gebrauch mit einem Oxidationsmittel und trägt eine für die Haarfärbebehandlung ausreichende Menge, je nach Haarfülle im allgemeinen etwa 60 bis 200 Gramm des erhaltenen gebrauchsfertigen Oxidationshaarfärbemittels auf das Haar auf.

Als Oxidationsmittel kommen hauptsächlich Wasserstoffperoxid oder dessen Additionsverbindungen an Harnstoff, Melamin oder Natriumbromat in Form einer 1- bis 12prozentigen, vorzugsweise 6prozentigen, wäßrigen Lösung in Betracht, wobei Wasserstoffperoxid besonders bevorzugt wird

Man läßt das erfindungsgemäße Haarfärbemittel bei 15 bis 50 Grad Celsius etwa 10 bis 45 Minuten, vorzugsweise 30 Minuten, lang auf das Haar einwirken, spült sodann das Haar mit Wasser und trocknet es. Gegebenenfalls wird im Anschluss an die Spülung mit einem Shampoo gewaschen und/oder mit einer schwachen organischen Säure, wie zum Beispiel Zitronensäure oder Weinsäure, nachgespült. Anschließend wird das Haar getrocknet.

Die nachfolgenden Beispiele sollen den Gegenstand näher erläutern, ohne ihn auf diese Beispiele zu beschränken:

## Beispiele

**Beispiel 1:** Haarfärbelösung

| | |
|---|---|
| 0,2 g | 2-(2',5'-Diaminophenyl)ethanol-sulfat |
| 0,1 g | 2-(2,4-Diaminophenoxy)ethanol-dihydrochlorid |
| 0,4 g | 2-Amino-6-chlor-4-nitrophenol |
| 10,0 g | Isopropanol |
| 10,0 g | Laurylalkohol-diglykolether-sulfat-Natriumsalz, 28prozentige wäßrige Lösung |
| 10,0 g | Ammoniak, 25prozentige wäßrige Lösung |
| 0,3 g | Ascorbinsäure |
| 69,0 g | Wasser, vollentsalzt |
| 100,0g | |

Zur Anwendung werden 10 g Haarfärbelösung mit 10 g Wasserstoffperoxid-Lösung (6prozentige wäßrige Lösung) gemischt. Das erhaltene Oxidationshaarfärbemittel wird auf durch Wind und Wetter geschädigte Haare aufgetragen. Nach einer Einwirkungszeit von 30 Minuten bei 40 °C werden die Haare mit Wasser gespült, schamponiert und getrocknet.

Das Haar hat einen gleichmäßig braunen Farbton angenommen.

**Beispiel 2:** Haarfärbecreme

| | |
|---|---|
| 0,3 g | 2-Hydroxymethyl-p-phenylendiamin-sulfat |
| 0,1 g | 1,3-Bis(2,4-Diaminophenoxy)propan-tetrahydrochlorid |
| 0,5 g | 2-Chlor-6-(ethylamino)-4-nitrophenol |
| 15,0 g | Cetylalkohol |
| 3,5 g | Laurylalkohol-diglykolether-sulfat-Natriumsalz, 28prozentige wäßrige Lösung |

(fortgesetzt)

| | |
|---|---|
| 3,0 g | Ammoniak, 25prozentige wäßrige Lösung |
| 0,3 g | Natrimsulfit, wasserfrei |
| 77,3 g | Wasser, vollentsalzt |
| 100,0 g | |

Zur Anwendung werden 10 g Haarfärbecreme mit 10 g Wasserstoffperoxid-Lösung (6prozentige wäßrige Lösung) gemischt. Das erhaltene Oxidationshaarfärbemittel wird auf durch Wind und Wetter geschädigte Haare aufgetragen. Nach einer Einwirkungszeit von 30 Minuten bei 40 °C werden die Haare mit Wasser gespült, schamponiert und getrocknet.

Das Haar ist gleichmäßig vom Ansatz bis zur Spitze in einem rotbraunen Ton gefärbt.

**Beispiel 3:** Haarfärbelösung

| | |
|---|---|
| 0,6 g | 2,5-Diaminoanisol |
| 0,6 g | 2-(2',4'-Diaminophenoxy)ethanol-dihydrochlorid |
| 0,2 g | 6-Chlor-2-((2'-hydroxyethyl)amino)-4-nitrophenol |
| 10,0 g | Isopropanol |
| 10,0 g | Laurylalkohol-diglykolether-sulfat-Natriumsalz, 28-prozentige wäßrige Lösung |
| 10,0 g | Ammoniak, 25prozentige wäßrige Lösung |
| 0,3 g | Ascorbinsäure |
| 68,3 g | Wasser, vollentsalzt |
| 100,0 g | |

Zur Anwendung werden 10 g Haarfärbelösung mit 10 g Wasserstoffperoxid-Lösung (6prozentige wäßrige Lösung) gemischt. Das erhaltene Oxidationshaarfärbemittel wird auf durch Wind und Wetter geschädigte Haare aufgetragen. Nach einer Einwirkungszeit von 30 Minuten bei 40 °C werden die Haare mit Wasser gespült, schamponiert und getrocknet.

Das Haar hat einen gleichmäßig schwarzen Farbton angenommen.

Die angegeben Prozentwerte stellen soweit nicht anders angegeben Gewichtsprozente dar.

## Patentansprüche

**1.** Haarfarbträgermasse enthaltend

1) - mindestens ein p-Phenylendiaminderivat, das ausgewählt ist aus
2-Hydroxymethyl-p-phenylendiamin,
2-(2',5'-Diaminophenyl)-ethanol und
2-(2'-Hydroxyethoxy)-p-phenylendiamin,
2) - mindestens ein m-Phenylendiaminderivat, das ausgewählt ist aus
2-(2',4'-Diaminophenoxy)ethanol,
1,3-Bis-(2',4'-diaminophenoxy)-propan,
Bis-(2',4'-diaminophenoxy)methan,
2-Amino-4-((2'-hydroxyethyl)-amino)-anisol,
1-(2'-Aminoethoxy)-2,4-diaminobenzol,
2-(2'-Hydroxyethoxy)-5-(methylamino)-anilin,
2,4-Diamino-1,5-bis-(2'-hydroxyethoxy)benzol
und 1-(2',3'-Dihydroxypropoxy)-2,4-diaminobenzol,
und
3) - mindestens ein 2-Amino-6-Chlor-4-nitrophenol der allgemeinen Formel (I),

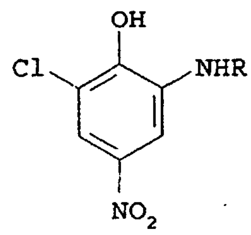

I

worin R Wasserstoff, eine geradkettigen oder verzweigte Alkylgruppe mit 1 bis 6 Kohlenstoffatomen, eine Hydroxyalkylgruppe mit 2 bis 4 Kohlenstoffatomen oder eine geradkettige oder verzweigte Polyhydroxyalkylgruppe mit 3 bis 4 Kohlenstoffatomen bedeutet,

**2.** Haarfarbträgermasse nach Anspruch 1, **dadurch gekennzeichnet, daß** die Verbindung der Formel (I) ausgewählt ist aus 2-Amino-6-chlor-4-nitrophenol, 6-Chlor-2-ethylamino-4-nitrophenol und 6-Chlor-2-((2'-hydroxyethyl)amino)-4-nitrophenol.

**3.** Haarfarbträgermasse nach Anspruch 1 oder 2, **dadurch gekennzeichnet, daß** zusätzlich weitere Oxidationsfarbvorstufen aus der Gruppe bestehend aus p-Phenylendiaminderivaten, Resorcinderivaten und 1,3-Benzodioxolderivaten enthalten sind.

**4.** Haarfarbträgermasse nach einem der Ansprüche 1 bis 3, **dadurch gekennzeichnet, daß** zusätzlich direktziehende Farbstoffe enthalten sind.

**5.** Haarfarbträgermasse nach einem der Ansprüche 1 bis 4, **dadurch gekennzeichnet, daß** die Farbvorstufen der erfindungsgemäßen Kombination in Form ihrer Additonssalze mit einer anorganischen oder organischen Säure enthalten sind.

**6.** Haarfarbträgermasse nach einem der Ansprüche 1 bis 5, **dadurch gekennzeichnet, daß** die Farbvorstufen in einem geeigneten kosmetischen Träger angewendet werden.

**7.** Haarfarbträgermasse nach einem der Ansprüche 1 bis 6, **dadurch gekennzeichnet, daß** die Farbvorstufen in ihr in einer Gesamtmenge von 0,1 bis 10 Gewichtsprozent enthalten sind.

**8.** Haarfarbträgermasse nach einem der Ansprüche 1 bis 7, **dadurch gekennzeichnet, daß** sie weitere übliche kosmetische Zusätze enthält.

**9.** Haarfärbemittel, **dadurch gekennzeichnet, daß** es durch Vermischen einer Haarfarbträgermasse nach einem der Ansprüche 1 bis 8 mit einem Oxidationsmittel unmittelbar vor der Anwendung hergestellt wird.

**10.** Haarfärbemittel nach Anspruch 9, **dadurch gekennzeichnet, daß** die Haarfarbträgermasse mit dem Oxidationsmittel in einem Verhältnis von 5:1 bis 1:3 vermischt wird.

**11.** Haarfärbemittel nach Anspruch 9 oder 10, dadurch gekenn zeichnet, daß es einen pH-Wert von 3 bis 11 aufweist.

**12.** Verfahren zum oxidativen Färben von Haaren, dadurch gekenn zeichnet, daß ein Haarfärbemittel nach einem der Ansprüche 9 bis 11 auf das Haar aufgetragen wird, bei einer Temperatur von 15 bis 50°C 10 bis 45 Minuten lang einwirken gelassen wird, das Haar anschließend mit Wasser gespült, gegebenenfalls schamponiert und sodann getrocknet wird.

## Revendications

**1.** Matière colorante pour cheveux, contenant

1) au moins un dérivé de p-phénylènediamine qui est choisi parmi
la 2-hydroxyméthyl-p-phénylènediamine,
le 2-(2',5'-diaminophényl)éthanol et
la 2-(2'-hydroxyéthoxy)-p-phénylènediamine,
2) au moins un dérivé de m-phénylènediamine qui est choisi parmi
le 2-(2',4'-diaminophénoxy)éthanol,
le 1,3-bis(2',4'-diaminophénoxy)propane,
le bis(2',4'-diaminophénoxy)méthane,
le 2-amino-4-((2'-hydroxyéthyl)amino)anisole,
le 1-(2'-aminoéthoxy)-2,4-diaminobenzène,
la 2-(2'-hydroxyéthoxy)-5-(méthylamino)aniline,
le 2,4-diamino-1,5-bis(2'-hydroxyéthoxy)benzène et
le 1-(2',3'-dihydroxypropoxy)-2,4-diaminobenzène,
et
3) au moins un 2-amino-6-chloro-4-nitrophénol correspondant à la formule générale (I),

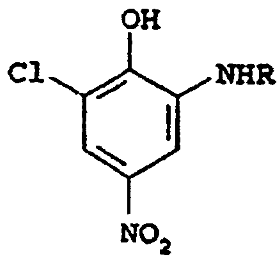

I

dans laquelle R représente un atome d'hydrogène, un groupe alkyle à chaîne droite ou ramifiée ayant de 1 à 6 atomes de carbone, un groupe hydroxyalkyle ayant de 2 à 4 atomes de carbone ou un groupe polyhydroxyalkyle à chaîne droite ou ramifiée ayant 3 ou 4 atomes de carbone.

**2.** Matière colorante pour cheveux selon la revendication 1, **caractérisée en ce que** le composé de formule (I) est choisi parmi le 2-amino-6-chloro-4-nitrophénol, le 6-chloro-2-éthylamino-4-nitrophénol et le 6-chloro-2-((2'-hydroxyéthyl)amino)-4-nitrophénol.

**3.** Matière colorante pour cheveux selon la revendication 1 ou 2, **caractérisée en ce qu'**elle contient en outre d'autres précurseurs de colorants d'oxydation choisis dans le groupe constitué par des dérivés de p-phénylènediamine, des dérivés de résorcinol et des dérivés de 1,3-benzodioxole.

**4.** Matière colorante pour cheveux selon l'une quelconque des revendications 1 à 3, **caractérisée en ce qu'**elle contient en outre des colorants directs.

**5.** Matière colorante pour cheveux selon l'une quelconque des revendications 1 à 4, **caractérisée en ce que** les précurseurs de colorants de l'association selon l'invention sont contenus sous forme de leurs sels d'addition avec un acide organique ou minéral.

**6.** Matière colorante pour cheveux selon l'une quelconque des revendications 1 à 5, **caractérisée en ce que** les précurseurs de colorants sont utilisés dans un véhicule cosmétique approprié.

**7.** Matière colorante pour cheveux selon l'une quelconque des revendications 1 à 6, **caractérisée en ce que** les précurseurs de colorants sont contenus dans celle-ci en une quantité totale de 0,1 à 10 % en poids.

**8.** Matière colorante pour cheveux selon l'une quelconque des revendications 1 à 7, **caractérisée en ce qu'**elle contient d'autres additifs cosmétiques usuels.

**9.** Produit de teinture pour cheveux, **caractérisé en ce qu'**il est préparé immédiatement avant l'emploi, par mélange

d'une matière colorante pour cheveux selon l'une quelconque des revendications 1 à 8, avec un oxydant.

**10.** Produit de teinture pour cheveux selon la revendication 9, **caractérisé en ce que** la matière colorante pour cheveux est mélangée avec l'oxydant en un rapport de 5:1 à 1:3.

**11.** Produit de teinture pour cheveux selon la revendication 9 ou 10, **caractérisé en ce qu'**il présente un pH de 3 à 11.

**12.** Procédé pour la teinture par oxydation des cheveux, **caractérisé en ce qu'**on applique sur les cheveux un produit de teinture pour cheveux selon l'une quelconque des revendications 9 à 11, on laisse agir pendant 10 à 45 minutes à une température de 15 à 50°C, puis on rince les cheveux à l'eau, éventuellement on les shampooine et ensuite on les sèche.

**Claims**

**1.** Hair colour carrier mass comprising

1) - at least one p-phenylenediamine derivative which is chosen from
2-hydroxymethyl-p-phenylenediamine,
2-(2',5' -diaminophenyl) ethanol and
2-(2'-hydroxyethoxy)-p-phenylenediamine,
2) - at least one m-phenylenediamine derivative which is chosen from
2- (2',4'-diaminophenoxy)ethanol,
1,3-bis(2',4'-diaminophenoxy)propane,
bis(2',4'-diaminophenoxy)methane,
2-amino-4-((2'-hydroxyethyl)amino)anisole,
1-(2'-aminoethoxy)-2,4-diaminobenzene,
2-(2'-hydroxyethoxy)-5-(methylamino)aniline,
2,4-diamino-1,5-bis(2'-hydroxyethoxy)benzene
and 1-(2',3'-dihydroxypropoxy)-2,4-diaminobenzene,
and
3) - at least one 2-amino-6-chloro-4-nitrophenol of the general formula (I),

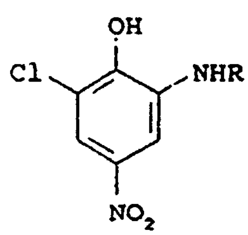

I

in which R is hydrogen, a straight-chain or branched alkyl group having 1 to 6 carbon atoms, a hydroxyalkyl group having 2 to 4 carbon atoms or a straight-chain or branched polyhydroxyalkyl group having 3 to 4 carbon atoms.

**2.** Hair colour carrier mass according to Claim 1, **characterized in that** the compound of the formula (I) is chosen from 2-amino-6-chloro-4-nitrophenol, 6-chloro-2-ethylamino-4-nitrophenol and 6-chloro-2-((2'-hydroxyethyl)amino)-4-nitrophenol.

**3.** Hair colour carrier mass according to Claim 1 or 2, **characterized in that** further oxidation dye precursors from the group consisting of p-phenylenediamine derivatives, resorcinol derivatives and 1,3-benzodioxol derivatives are additionally present.

**4.** Hair colour carrier mass according to one of Claims 1 to 3, **characterized in that** direct dyes are additionally present.

5. Hair colour carrier mass according to one of Claims 1 to 4, **characterized in that** the dye precursors of the combination according to the invention are present in the form of their addition salts with an inorganic or organic acid.

6. Hair colour carrier mass according to one of Claims 1 to 5, **characterized in that** the dye precursors are used in a suitable cosmetic carrier.

7. Hair colour carrier mass according to one of Claims 1 to 6, **characterized in that** the dye precursors are present therein in a total amount of from 0.1 to 10% by weight.

8. Hair colour carrier mass according to one of Claims 1 to 7, **characterized in that** it comprises further customary cosmetic additives.

9. Hair dyeing agent **characterized in that** it is prepared by mixing a hair colour carrier mass according to one of Claims 1 to 8 with an oxidizing agent directly prior to use.

10. Hair dyeing agent according to Claim 9, **characterized in that** the hair colour carrier mass is mixed with the oxidizing agent in a ratio of from 5:1 to 1:3.

11. Hair dyeing agent according to Claim 9 or 10, **characterized in that** it has a pH of from 3 to 11.

12. Method for the oxidative dyeing of hair, **characterized in that** the hair dyeing agent according to one of Claims 9 to 11 is applied to the hair, left to act at a temperature of from 15 to 50°C for 10 to 45 minutes, the hair is then rinsed with water, optionally shampooed and then dried.